(19) Europäisches Patentamt
European Patent Office
Office européen des brevets

(11) Publication number : **0 671 393 A1**

# (12) EUROPEAN PATENT APPLICATION

(21) Application number : **95301483.4**

(22) Date of filing : **07.03.95**

(51) Int. Cl.[6] : **C07D 241/46,** C07D 403/04, G03C 1/498, C09B 17/00, C09B 17/02

(30) Priority : **11.03.94 GB 9404806**

(43) Date of publication of application : **13.09.95 Bulletin 95/37**

(84) Designated Contracting States : **DE FR GB IT**

(71) Applicant : **MINNESOTA MINING AND MANUFACTURING COMPANY**
**3M Center,**
**P.O. Box 33427**
**St. Paul, Minnesota 55133-3427 (US)**

(72) Inventor : **Grieve, Duncan McL. A.**
**10 Churchfield**
**Saffron Walden, Essex CB11 4BG (GB)**
Inventor : **Mott, Andrew W.**
**50 Portland Road**
**Bishops Stortford, Hertfordshire (GB)**
Inventor : **Nairne, Robert J.D.**
**c/o Minnesota 3M Res. Ltd.,**
**Pinnacles**
**Harlow, Essex CM19 5AE (GB)**
Inventor : **Bays, David C.**
**26 Danbury Road**
**Loughton, Essex IG10 3AP (GB)**
Inventor : **Poon, Stephen S.C.**
**c/o Minnesota 3M Res. Ltd.,**
**Pinnacles**
**Harlow, Essex CM19 5AE (GB)**
Inventor : **Attwood, Martin D.**
**16 Greygoose Park**
**Harlow, Essex CM19 5NN (GB)**
Inventor : **Jackson, Andrew C.**
**25 Walton Park,**
**Pannal**
**Harrogate. North Yorkshire HG3 1EJ (GB)**

(74) Representative : **Bowman, Paul Alan et al**
**LLOYD WISE, TREGEAR & CO.**
**Norman House**
**105-109 Strand**
**London WC2R OAE (GB)**

## (54) Monoaminophenazine leuco dyes and photothermographic materials containing same.

(57) Photothermographic elements incorporating leuco forms of phenazinium dyes to provide a developed colour image. The dye has a nucleaus of the general formula :

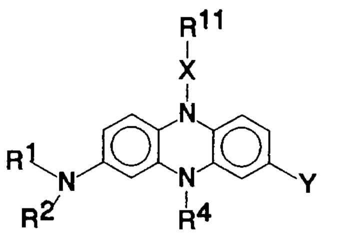

(I)

in which ;

X represents

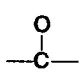 , 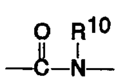 , 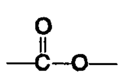 or 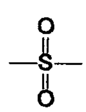

Y is H or any substituent other than amino or amido,

$R^1$, $R^2$, $R^4$ and $R^{10}$ are independently selected from H, alkyl, aryl, alkylsulfonyl, arysulfonyl, alkylcarbonyl, arylcarbonyl, or

$R^1$ and $R^2$ together represent the necessary atoms to complete a cyclic structure or one or more of $R^1$, $R^2$ and $R^4$ represent the necessary atoms to complete a cyclic structure fused to the nucleus having skeletal atoms selected from C, N, 0 and S, and

EP 0 671 393 A1

$R^{11}$ represents any group which will not prevent oxidative cleavage of the X-N bond, with the proviso that when $R^1$ is $C_2H_5$
$R^2$ is not $C_2H_4NHSO_2CH_3$.

This invention relates to leuco forms of phenazinium dyes and to photothermographic elements, compositions and processes which incorporate these leuco dyes to provide a developed imaqe in colour.

3,7-bis-amino-5-aryl or -alkyl phenazinium dyes, commonly called safranines have been known for many years and used for a variety of purposes. In addition the leuco forms of these dyes have found use in imaging systems including photothermographic elements. 3-Amino-5-aryl or -alkyl phenazinium dyes, the aposafranines, have been used less extensively than their bis-amino counterparts and there are few reports of leuco aposafranines.

US-A-4563415 discloses thermographic systems using naphthoylated leuco phenazine dyes in reactive association with nitrate salts. Whilst the general formula extends to both safranines and aposafranines all of the specific dyes disclosed are safranines.

FR-A-2113509 discloses a range of leuco dyes with a general formula covering dyes having an azine, oxazine and thiazine nucleus. All of the phenazine dyes disclosed are 3,7-diamino substituted.

EP-A-0302610 discloses light sensitive, dry processing imaging materials substantially free from photosensitive silver which may comprise a leuco dye in combination with a photosensitive compound such as an onium salt. Suitable leuco dyes include acylated azine, phenoxazine and phenothiazine. There is a single disclosure of a magenta leuco dye by structural formula as an aposafranine which appears to be an error and should have disclosed a safranine since the general formula of the leuco dyes does not extend to aposafranines and all the other relevant dyes disclosed are safranines.

Japanese Patent Application No. 03-153234 discloses specifically Compound 28 on page 6 of the published application. This compound appears in a list of over 30 compounds claimed to be useful in wet-developed silver halide emulsions for improving the tone of a developed silver image.

According to the present invention there is provided a leuco dye having a nucleus of the general formula:

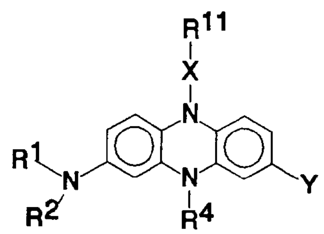

(I)

in which;

X represents

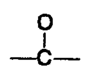 , 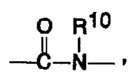 , 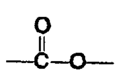 or 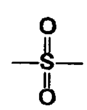

Y is H or any substituent other than amino or amido,

$R^1$, $R^2$, $R^4$ and $R^{10}$ are independently selected from H, alkyl, aryl, alkylsulfonyl, arylsulfonyl, alkylcarbonyl, arylcarbonyl, or

$R^1$ and $R^2$ together represent the necessary atoms to complete a cyclic structure or one or more of $R^1$, $R^2$ and $R^4$ represent the necessary atoms to complete a cyclic structure fused to the nucleus having skeletal atoms selected from C, N, 0 and S, and

$R^{11}$ represents any group which will not prevent oxidative cleavage of the X-N bond,

with the proviso that when $R^1$ is $C_2H_5$ $R^2$ is not $C_2H_4NHCO_2CH_3$.

The dyes are preferably free of amine substituents on the rings other than the $-NR^1R^2$ group.

This invention provides a new class of novel leuco phenazinium dyes where the 3 position of the phenazine contains a substituted or unsubstituted amino group and the 7 position is unsubstituted or contains a substituent other than nitrogen. These leuco dyes can be used to form dyes (the oxidised form) capable of providing yellow, orange, red, magenta, purple and blue colours. The leuco dyes of the invention have improved stability to oxidation as compared to their 3,7-bis-amino substituted counterparts described in the prior art.

The leuco dyes of this invention can be employed in a photothermographic material comprising a combination of a) photographic silver halide b) a silver salt of a long chain fatty acid c) the leuco dye and d) a polymer

binder for the combination where the leuco dye is oxidisable imagewise by the Ag salt in a thermal process catalysed by light-exposed silver halide, to release the corresponding phenazinium dye. In the absence of the leuco dye no desired colour image is developed upon heating the combination of components.

The combination of components containing the leuco dyes of this invention can be employed in a diffusion transfer photothermographic material comprising a support having coated thereon i) a layer comprising: a) photographic silver halide in association with b) a silver salt of a long chain fatty acid c) the leuco dye of this invention and d) a polymeric binder, and ii) an image receiving layer with is capable of receiving the phenazinium dye formed in the described layer i).

The leuco phenazinium dyes of this invention are generally of the formula:

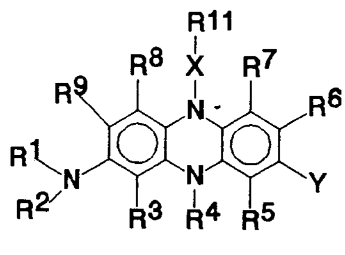

(II)

in which;

$R^1$, $R^2$, $R^4$, $R^{11}$, X and Y are as defined above,

$R^3$ and $R^5$ to $R^9$ are selected from H, alkyl, aryl, heterocyclic, alkoxy, aryloxy, amino, cyano, hydroxy, halogen, nitro, mercapto, alkylthio, arylthio, alkylsuphonyl, arylsulphonyl, alkylcarbonyl, arylcarbonyl, acyloxy, amido, sulphonamido and one or more of $R^2$ and $R^3$, $R^3$ and $R^4$ , $R^4$ and $R^5$, $R^5$ and Y, Y and $R^6$, $R^6$ and $R^7$, $R^8$ and $R^9$, or $R^9$ and $R^1$ may, together with the intervening skeletal atoms, complete a cyclic structure wherein the ring atoms are selected from C, N, O and S,

Y may represent any substituent other than amino or amido and is preferably selected from those ranges of groups specified for $R^3$ and $R^5$ to $R^9$ with the exception of amino or amido groups,

$R^{11}$ may represent any organic group ranging from alkyl, aryl, heterocyclic, polymeric chain etc. $R^{11}$ advantageously forms part of a multifunctional linking group to which two or more moieties of formula (I) or formula II) are attached as disclosed in our copending application of even date.

The term "heterocyclic" refers to both aromatic and non-aromatic rings wherein the atoms are chosen from C, N, O and S; "aryl" refers to aromatic ring systems comprising up to 14 carbons; and "alkyl" refers to aliphatic residues (linear or cyclic) comprising up to 20 carbon atoms. Alkyl, aryl and heterocyclic groups represented by $R^1$ to $R^{11}$ may bear further substituents such as those listed for $R^1$ to $R^{11}$.

Oxidation of the leuco dyes (I) and (II) results in cleavage of the X-N bond releasing the corresponding phenazinium dyes I(a) and II(a), where Y and $R^1$ to $R^9$ have the same meaning as before:

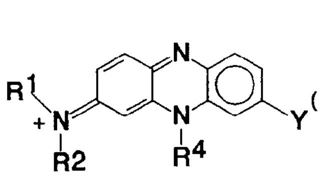

(Ia) or

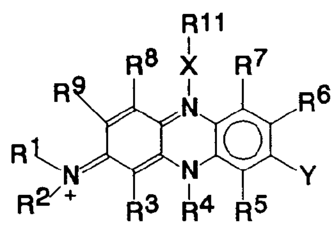

(IIa)

In diffusion-transfer imaging elements based on compounds (I) and (II), it is necessary for the dyes (Ia) and (IIa) to diffuse thermally to a separate receptor layer. Hence substituents Y and $R^1$ to $R^9$ may be chosen so as to be compatible with this diffusion, and so smaller alkyl and aryl residues e.g. up to 5 and 6 carbon atoms respectively, and the absence of bulky or highly polar substituents will normally be preferred. Conversely, in order to restrict the diffusibility of compounds (I) and (II), it may be desirable for the moiety $R^{11}$ to incorporate bulky and/or polar groups.

Because these leuco dyes are essentially colourless, they provide a means of producing an image when the leuco is oxidised. A colour image is intended to mean an image which is other than colourless and includes images which can be observed within the visible portion of the spectrum. It also includes images which can be observed in other parts of the spectrum such as the ultra violet portion of the spectrum. Typically the coloured image from the leuco dyes of this invention is yellow or magenta. The desired colour of the image can be predetermined by selection of the substituents $R^1$ to $R^9$ and Y of the leuco dye.

The described leuco dyes can be prepared from their corresponding dyes by methods known in the art for the reduction of other azine dyes. For example, U.S.A. Patent No. 4,622,395 describes the preparation of leuco phenothiazine and phenoxazine dyes. The aposafranine dyes from which the leuco dyes of this invention may be prepared are described in the literature (VS Mokrushin, Z V Pushkareva Khim Farm Zh 5, 21 (1969)) and are prepared from phenazinium salts and amines in the presence of air. A preferred preparation of these aposafranines described herein involves reaction of 3-chlorophenazinium salts with amines where the chlorine is displaced by the amine in good yield.

One embodiment of this invention is a photothermographic element for producing colour images comprising a support having coated thereon a) photographic silver halide in association with b) a silver salt of a long chain fatty acid, c) a leuco dye of this invention and d) a polymer binder.

It is believed that the latent image silver from the photographic silver halide acts as a catalyst for the reaction between the silver salt of a long-chain fatty acid and the described leuco dye reducing agent. The term "in association with" as employed herein regarding described photosensitive silver halide is intended to mean that the location of the photosensitive silver halide in the photothermographic element or composition of the invention is such that will enable this catalysis.

Thus, the photothermographic dry silver emulsions of this invention may be constructed of one or more layers on a substrate. Single layer constructions must contain the silver source material, the silver halide, the developer and binder as well as optional additional materials such as toners, coating aids, and other adjuvants. Two-layer constructions must contain the silver source and silver halide in one emulsion layer (usually the layer adjacent to the substrate) and some of the other ingredients in the second layer or both layers, although two layer constructions comprising a single emulsion layer containing all the ingredients and a protective topcoat are envisioned. Multicolour photothermographic dry silver constructions may contain sets of these bilayers for each colour, or they may contain all ingredients within a single layer as described in U.S. Patent No. 4,708,928. In the case of multilayer multicolour photothermographic articles the various emulsion layers are generally maintained distinct from each other by the use of functional or non-functional barrier layers between the various photosensitive layers as described in U.S. Patent No. 4,460,681.

While not necessary for practice of the present invention, it may be advantageous to add mercury (II) salts to the emulsion layer(s) as an antifoggant. Preferred mercury (II) salts for this purpose are mercuric acetate and mercuric bromide.

The light sensitive silver halide used in the present invention may typically be employed in a range of 0.75 to 50 mol percent and, preferably, from 2 to 20 mol percent of organic silver salt.

The silver halide may be any photosensitive silver halide such as silver bromide, silver iodide, silver chloride, silver bromoiodide, silver chlorobromoiodide, silver chlorobromide, etc. The silver halide may be in any form which is photosensitive including, but not limited to cubic, orthorhombic, tabular, tetrahedral, etc., and may have epitaxial growth of crystals thereon. Essentially any grain size or mixture of grain sizes in the range to 0.001 to 1.0 micron may be used, but very fine grains e.g. of 0.05 micron of low dispersity are preferred.

The silver halide used in the present invention may be employed without modification. However, it may be chemically sensitized with a chemical sensitizing agent such as a compound containing sulphur, selenium or tellurium etc., or a compound containing gold, platinum, palladium, rhodium or iridium, etc., a reducing agent such as a tin halide, etc., or a combination thereof. The details of these procedures are described in T.N. James "The Theory of the Photographic Process", Fourth Edition, Chapter 5, pages 149 to 169.

The silver halide may be added to the emulsion layer in any fashion which places it in catalytic proximity to the silver source. Silver halide and the organic silver salt which are separately formed or "preformed" in a binder can be mixed prior to use to prepare a coating solution, but it is also effective to blend both of them in a ball mill for a long period of time. Further, it is effective to use a process which comprises adding a halogen-containing compound in the organic silver salt prepared to partially convert the silver of the organic silver salt to silver halide.

Methods of preparing these silver halide and organic silver salts and manners of blending them are known in the art and described in *Research Disclosure*, June 1978, item 17029, and U.S. Patent No. 3,700,458.

The preformed silver halide emulsions of this invention can be unwashed or washed to remove soluble salts. In the latter case the soluble salts can be removed by chill-setting and leaching or the emulsion can be coagulation washed, e.g., by the procedures described in U.S. Patents Nos. 2,618,556; 2,614,928; 2,565,418; 3,241,969; and 2,489,341. The silver halide grains may have any crystalline habit including, but not limited to cubic, tetrahedral, orthorhombic, tabular, laminar, platelet, etc.

Silver halide emulsions can be protected against the additional production of fog and can be stabilized against loss of sensitivity during shelf storage. Suitable antifoggants, stabilizers, and stabilizer precursors

which can be used alone or in combination, include thiazolium salts as described in U.S. Patents Nos. 2,131,038 and 2,694,716; azaindenes as described in U.S. Patents Nos. 2,886,437 and 2,444,605; mercury salts as described in U.S. Patent No. 2,728,663; urazoles as described in U.S. Patent No. 3,287,135; sulfocatechols as described in U.S. Patent No. 3,235,652; oximes as described in British Patent No. 623,448; nitrones; nitroindazoles; polyvalent metal salts as described in U.S. Patent No. 2,839,405; thiouronium salts as described in U.S. Patent No. 3,220,839; and palladium, platinum and gold salts described in U.S. Patents Nos. 2,566,263 and 2,597,915; halogen-substituted organic compounds as described in U.S. Patents Nos. 4,108,665 and 4,442,202; triazines as described in U.S. Patents Nos. 4,128,557; 4,137,079; 4,138,265; and 4,459,350; and phosphorous compounds as described in U.S. Patent No. 4,411,985.

Emulsions used in the invention can contain plasticisers and lubricants such as polyalcohols (e.g., glycerin and diols of the type described in U.S. Patent No. 2,960,404); fatty acids or esters such as those described in U.S. Patent No. 2,588,765 and U.S. Patent No. 3,121,060; and silicone resins such as those described in British Patent No. 955,061.

The organic silver salt may be any organic material which contains a reducible source of silver ions. Silver salts of organic acids, particularly long chain (10 to 30 preferably 15 to 28 carbon atoms) fatty carboxylic acids are preferred. Complexes of organic or inorganic silver salts wherein the ligand has a gross stability constant between 4.0 and 10.0 are also desirable. The silver source material generally constitutes from about 20 to 70 percent by weight of the imaging layer, preferably 30 to 55 percent.

The organic silver salt which can be used in the present invention is a silver salt which is comparatively stable to light, but forms a silver image when heated to 80 °C or higher in the presence of an exposed photocatalyst (such as photographic silver halide) and a reducing agent.

Preferred organic silver salts include silver salts of organic compounds having a carboxy group. Non-limiting examples thereof include silver salts of an aliphatic carboxylic acid and a silver salt of an aromatic carboxylic acid. Preferred examples of the silver salts of aliphatic carboxylic acids include silver behenate, silver stearate, silver oleate, silver laurate, silver caproate, silver myristate, silver palmitate, silver maleate, silver fumarate, silver tartrate, silver linoleate, silver butyrate and silver camphorate, mixtures thereof, etc. Silver salts with a halogen atom or a hydroxyl on the aliphatic carboxylic acid can also be effectively used. Preferred examples of the silver salts of aromatic carboxylic acids and other carboxyl group-containing compounds include silver benzoate, a silver substituted benzoate such as silver 3,5-dihydroxybenzoate, silver *o*-methylbenzoate, silver *m*-methylbenzoate, silver *p*-methylbenzoate, silver 2,4-dichlorobenzoate, silver acetamidobenzoate, silver *p*-phenyl benzoate, etc., silver gallate, silver tannate, silver phthalate, silver terephthalate, silver salicylate, silver phenylacetate, silver pyromellitate, a silver salt of 3-carboxymethyl-4-methyl-4-thiazoline-2-thione or the like as described in U.S. Patent No. 3,785,830, and silver salt of an aliphatic carboxylic acid containing a thioether group as described in U.S. Patent No. 3,330,663, etc.

Silver salts of compounds containing mercapto or thione groups and derivatives thereof can also be used. Preferred examples of these compounds include a silver salt of 3-mercapto-4-phenyl-l,2,4-triazole, a silver salt of 2-mercaptobenzimidazole, a silver salt of 2-mercapto-5-aminothiadiazole, a silver salt of 2-(ethylglycolamido) benzothiazole, a silver salt of thioglycolic acid such as a silver salt of an S-alkyl thioglycolic acid (wherein the alkyl group has from 12 to 22 carbon atoms), a silver salt of a dithiocarboxylic acid such as a silver salt of dithioacetic acid, a silver salt of a thioamide, a silver salt of 5-carboxylic-1-methyl-2-phenyl-4-thiopyridine, a silver salt of mercaptotriazine, a silver salt of 2-mercaptobenzoxazole, a silver salt as described in U.S. Patent No. 4,123,274, for example, a silver salt of l,2,4-mercaptothiazole derivative such as a silver salt of 3-amino-5-benzylthio-1,2,4-thiazole, a silver salt of thione compound such as a silver salt of 3-(2-carboxyethyl)-4-methyl-4-thiazoline-2-thione as disclosed in U.S. Patent No. 3,301,678.

Furthermore, a silver salt of a compound containing an imino group may be used. Preferred examples of these compounds include silver salts of benzothiazole and derivatives thereof, for example, silver salts of benzothiazoles such as silver methylbenzotriazolate, etc., silver salt of halogen-substituted benzotriazoles, such as silver 5-chlorobenzotriazolate, etc., silver salts of carboimidobenzotriazole, etc., silver salt of l,2,4-triazoles or l-*H*-tetrazoles as described in U.S Patent No. 4,220,709, silver salts of imidazoles and imidazole derivatives, and the like. Various silver acetylide compounds can also be used, for instance, as described in U.S. Patents Nos. 4,761,361 and 4,775,613.

It is also found convenient to use silver half soaps, of which an equimolar blend of silver behenate and behenic acid, prepared by precipitation from aqueous solution of the sodium salt of commercial behenic acid and analysing about 14.5 percent silver, represents a preferred example. Transparent sheet materials made on transparent film backing require a transparent coating and for this purpose the silver behenate full soap, containing not more than about four or five percent of free behenic acid and analysing about 25.2 percent silver may be used.

The method used for making silver soap dispersions is well known in the art and is disclosed in *Research*

*Disclosure*, April 1983, item 22812, *Research Disclosure*, October 1983, item 23419 and U.S. Patent No. 3,985,565.

The light-sensitive silver halides may be advantageously spectrally sensitized with various known dyes including cyanine, merocyanine, styryl, hemicyanine, oxonol, hemioxonol and xanthene dyes. Useful cyanine dyes include those having a basic nucleus, such as a thiazoline nucleus, an oxazoline nucleus, a pyrroline nucleus, a pyridine nucleus, an oxazole nucleus, a thiazole nucleus, a selenazole nucleus and an imidazole nucleus. Useful merocyanine dyes which are preferred include those having not only the above described basic nuclei but also acid nuclei, such as a thiohydantoin nucleus, a rhodanine nucleus, an oxazolidinedione nucleus, a thiazolidinedione nucleus, a barbituric acid nucleus, a thiazolinone nucleus, a malononitrile nucleus and a pyrazolone nucleus. In the above described cyanine and merocyanine dyes, those having imino groups or carboxyl groups are particularly effective. Practically, the sensitizing dyes to be used in the present invention may be properly selected from known dyes such as those described in U.S. Patents Nos. 3,761,279, 3,719,495, and 3,877,943, British Patents Nos. 1,466,201, 1,469,117 and 1,422,057, and can be located in the vicinity of the photocatalyst according to known methods. Spectral sensitizing dyes may be typically used in amounts of about $10^{-4}$ mol to about 1 mol per 1 mol of silver halide.

In addition to the aforementioned ingredients it may be advantageous to include additives known as "toners" that improve the image. Toner materials may be present, for example, in amounts from 0.1 to 10 percent by weight of all silver bearing components. Toners are well known materials in the photothermographic art as shown in U.S. Patents Nos. 3,080,254; 3,847,612 and 4,123,282.

Examples of toners include phthalimide and N-hydroxyphthalimide; cyclic imides such as succinimide, pyrazoline-5-ones, and a quinazolinone, 3-phenyl-2-pyrazoline-5-one, 1-phenylurazole, quinazoline, and 2,4-thiazolidinedione; naphthalimides (e.g., *N*-hydroxy-l,8-naphthalimide); cobalt complexes (e.g., cobaltic hexamine trifluoroacetate); mercaptans as illustrated by 3-mercapto-l,2,4-triazole, 2,4-dimercaptopyrimidine, 3-mercapto-4,5-diphenyl-l,2,4-triazole and 2,5-dimercapto-1,3,4-thiadiazole; *N*-(aminomethyl) aryldicarboximides, (e.g., (*N,N*-dimethylaminomethyl)phthalimide, and *N,N*-(dimethylaminomethyl) naphthalene-2,3-dicarboximide); and a combination of blocked pyrazoles, isothiuronium derivatives and certain photobleaching agents (e.g., a combination of *N,N*'-hexamethylene bis(l-carbamoyl-3,5-dimethylpyrazole), l,8-(3,6-diazaoctane)bis(isothiuronium trifluoroacetate) and 2-(tribromomethylsulfonyl)benzothiazole); and merocyanine dyes such as 3-ethyl-5[(3-ethyl-2-benzo thiazolinylidene)-1-methylethylidene]-2-thio-2,4-oxazolidinedione; phthalazine and phthalazinone derivatives (or metal salts of these derivatives) such as 4-(l-naphthyl)phthalazinone, 6-chlorophthalazinone, 5,7-dimethoxyphthalazinone, and 2,3-dihydro-l,4 phthalazinedione; a combination of phthalazinone plus phthalic acid derivatives (e.g., phthalic acid, 4-methylphthalic acid, 4-nitrophthalic acid, and tetrachlorophthalic anhydride); quinazolinediones, benzoxazine or naphthoxazine derivatives; rhodium complexes functioning not only as tone modifiers, but also as sources of halide ion for silver halide formation *in situ*, such as ammonium hexachlororhodate (III), rhodium bromide, rhodium nitrate and potassium hexachlororhodate (III); inorganic peroxides and persulfates (e.g., ammonium peroxydisulfate and hydrogen peroxide); benzoxazine-2,4-diones such as l,3-benzoxazine-2,4-dione, 8-methyl-1,3-benzoxazine-2,4-dione, and 6-nitro-l,3-benzoxazine-2,4-dione; pyrimidines and asymmetric triazines (e.g., 2,4-dihydroxypyrimidine, 2-hydroxy-4-aminopyrimidine), azauracils, and tetrazapentalene derivatives (e.g, 3,6-dimercapto-1,4-diphenyl-l*H*,4*H*-2,3*a*,5,6*a*-tetrazapentalene, and l,4-di(*o*-chlorophenyl)-3,6-dimercapto-l*H*,4*H*-2,3*a*,5,6*a*-tet razapentalene).

The photothermographic element or composition as described can contain various synthetic polymeric binders alone or in combination as vehicles or binding agents and in various layers. Suitable materials are typically hydrophobic, but hydrophillic materials can be employed. They are transparent or translucent and include such naturally occurring substances as cellulose derivatives and synthetic polymeric substances such as polyvinyl compounds which are compatible with the described components of the photothermographic elements and compositions of the invention. Other synthetic polymeric materials which can be employed include dispersed vinyl compounds such as in latex form and particularly those which increase dimensional stability of photographic materials. Effective polymers include water insoluble polymers of alkyl acrylates and methacrylates, acrylic acid, sulfoalkylacrylates or methacrylates, and those which have cross-linking sites which facilitate hardening or curing as well as those which have recurring sulfobetaine units as described in Canadian Patent No. 774,054. Useful high molecular weight materials and resins include poly(vinyl butyral), cellulose acetate butyrate, polymethyl-methacrylate, ethyl cellulose, polystyrene, polyvinylchloride, chlorinated rubber, polyisobutylene, butadiene-styrene copolymers, vinyl chloride-vinyl acetate etc.

Optionally, these polymers may be used in combinations of two or more thereof. Such a polymer is used in an amount sufficient to carry the components dispersed therein, that is, within the effective range of the action as the binder. The effective range can be appropriately determined by one skilled in the art. As a guide in the case of carrying at least an organic silver salt, it can be said that a preferable ratio of the binder to the

organic silver salt ranges from 15:1 to 1:2, and particularly from 8:1 to 1:1.

The formulation for the photothermographic emulsion layer can be prepared by dissolving the photosensitive silver halide, the source of reducible silver, the leuco dye, optional additives, and the binder in an inert organic solvent, such as, for example, acetone, 2-butanone or tetrahydrofuran.

The formulation can be coated onto a support by methods well known in the art, such as, for example, wire-wound rod, knife, or extrusion coating. Typical wet thickness of the emulsion layer can range from about 10 to 100 micrometers (μm), and the layer can be dried in forced air at temperatures ranging from 20°C to 100°C. It is preferred that the thickness of the layer be selected to provide maximum image densities greater than 0.2, and more preferably in the range 0.5 to 2.5, as measured by a MacBeth Colour Densitometer Model TD 504 using the colour filter complementary to the dye colour.

Alternatively, the formulation may be spray dried to produce solid particles, which can then be redispersed in a second, possibly different, binder and then coated onto the support.

The formulation for the emulsion layer can also include coating aids such as fluoroaliphatic polyesters.

The support or substrate of the photothermographic element can be selected from a wide range of materials, including paper, glass, metal, polymeric film and the like, depending upon the particular imaging requirement. Preferred materials for the support include polymers having good heat stability, such as polyesters. A particularly preferred polyester is polyethylene terephthalate.

Barrier layers, preferably comprising a polymeric material, can also be present in the photothermographic element of the present invention. Polymers for the material of the barrier layer can be selected from natural and synthetic polymers such as gelatin, polyvinylalcohols, polyacrylic acids, sulfonated polystyrene, and the like. The polymers can optionally be blended with barrier aids such as silica.

Images derived from the photothermographic element are advantageously transferred to an image receiving layer in order to separate them from the associated black silver image, but this is not essential.

The image receiving layer of this invention can be any flexible or rigid, transparent layer made of thermoplastic polymer. The image receiving layer preferably has a thickness of at least 0.1 micrometer, more preferably from about 1 to about 10 micrometers, and a glass transition temperature of from about 20°C to about 200°C. In the present invention, any thermoplastic polymer or combination of polymers can be used, provided the polymer is capable of absorbing and fixing the dye. Because the polymer acts as a dye mordant, no additional fixing agents are required. Thermoplastic polymers that can be used to prepare the image receiving layer include polyesters, such as polyethylene terephthalates; polyolefins, such as polyethylene; cellulosics, such as cellulose acetate, cellulose butyrate, cellulose propionate; polystyrene; polyvinyl chloride; polyvinylidine chloride; polyvinyl acetate; copolymer of vinylchloride-vinylacetate; copolymer of vinylidene chloride-acrylonitrile; copolymer of styreneacrylonitrile; and the like.

The optical density of the dye image and even the actual colour of the dye image in the image receiving layer is very much dependent on the characteristics of the polymer of the image receiving layer, which acts as a dye mordant, and, as such, is capable of absorbing and fixing the dyes. A dye image having a reflection optical density in the range of from 0.3 to 3.5 (preferably from 1.5 to 3.5) or a transmission optical density in the range of from 0.2 to 2.5 (preferably from 1.0 to 2.5) can be obtained with the present invention.

The image receiving layer can be formed by dissolving at least one thermoplastic polymer in an organic solvent (e.g., 2-butanone, acetone, tetrahydrofuran) and applying the resulting solution to a support base or substrate by various coating methods known in the art, such as curtain coating, extrusion coating, dip coating, air-knife coating, hopper coating, and any other coating method used for coating solutions. After the solution is coated, the image receiving layer is dried (e.g. in an oven) to drive off the solvent. The image receiving layer may be strippably adhered to the photothermographic element. Strippable image receiving layers are described in U.S. Patent No. 4,594,307, incorporated herein by reference.

Selection of the binder and solvent to be used in preparing the emulsion layer significantly affects the strippability of the image receiving layer from the photosensitive element. Preferably, the binder for the image receiving layer is impermeable to the solvent used for coating the emulsion layer and is incompatible with the binder used for the emulsion layer. The selection of the preferred binders and solvents results in weak adhesion between the emulsion layer and the image receiving layer and promotes good strippability of the emulsion layer.

The photothermographic element can also include coating additives to improve the strippability of the emulsion layer. For example, fluoroaliphatic polyesters dissolved in ethyl acetate can be added in an amount of from about 0.02 to about 0.5 weight percent of the emulsion layer, preferably from about 0.1 to about 0.3 weight percent. A representative example of such a fluoroaliphatic polyester is FLUORAD FC 431, commercially available from Minnesota Mining and Manufacturing Company. Alternatively, a coating additive can be added to the image receiving layer in the same weight range to enhance strippability. No solvents need to be used in the stripping process. The strippable layer preferably has a delaminating resistance of 1 to 50g/cm

and a tensile strength at break greater than, preferably at least two times greater than, its delaminating resistance.

Preferably, the image receiving layer is adjacent to the emulsion layer to facilitate transfer of the dye that forms after the imagewise exposed emulsion layer is subjected to thermal development, for example, in a heated shoe and roller type heat processor.

In another embodiment, the coloured dye released in the emulsion layer can be transferred onto a separately coated image receiving sheet by placing the exposed emulsion layer in intimate face to face contact with the image receiving sheet and heating the resulting composite construction. Good results can be achieved in this second embodiment when the layers are in uniform contact for a period of time of from 0.5 to 300 seconds at a temperature of from about 80°C to about 220°C.

Multi-colour images can be prepared by superimposing in register, imaged image receiving layers as prepared above. The polymers of the individual imaged image receiving layers must be sufficiently adherent to provide useful multi-coloured reproduction on a single substrate.

Alternatively, multi-colour images can be produced from a photothermographic element comprising a substrate and two or more photothermographic layers capable of forming different colours in response to light exposure and thermal development, said photothermographic layers being sensitive to different wavelengths of light, and at least one of said photothermographic layers comprising a leuco dye of Formula I as the colour forming agent. Preferably the element also comprises an image receiving layer and colour is formed in the photothermographic layers in the form of diffusible dyes. The various layers may be coated sequentially by the methods outlined above, optionally with barrier layers separating the light sensitive layers in order to minimise migration of active ingredients among the layers, which would lead to crosstalk.

Another method of minimising crosstalk between the layers is to coat each light sensitive layer on a separate carrier sheet and laminate them sequentially on the final substrate, i.e. on top of the image receiving layer if one is used. Subsequent to the lamination of each light sensitive layer, its associated carrier sheet is peeled off and discarded before lamination of the next layer. Lamination is readily carried out using a heated roller device, such as MATCHPRINT Laminator, with roller temperatures in the range 100 to 275°F. Suitable materials for the carrier sheets include polyester of thickness 25 to 200 microns, optionally treated with a release agent such as FLUORAD FC 431 to facilitate peeling.

Development conditions will vary, depending on the construction used, but will typically involve heating the imagewise exposed material at a suitably elevated temperature, e.g. from about 80°C to about 250°C, preferably from about 120°C to about 200°C, for a sufficient period of time, generally from 1 second to 2 minutes.

In some methods, the development is carried out in two steps. Thermal development takes place at a higher temperature, e.g. about 150°C for about 10 seconds, followed by thermal diffusion at a lower temperature, e.g. 80°C, optionally in the presence of a transfer solvent. The second heating step at the lower temperature prevents further development and allows the dyes that are already formed to diffuse out of the emulsion layer(s).

The material of this invention can be used for example, in conventional colour photography, in electronically generated colour hardcopy recording, and in digital colour proofing in the graphic arts area. The material of this invention provides high photographic speed, provides pure dye images, and provides a dry and rapid process.

The invention will now be illustrated by the following Examples.

Glossary of materials used:-

| | |
|---|---|
| VYNS | - copolymer of vinyl chloride/vinyl acetate supplied by Union Carbide. |
| FC431 | - Fluorad FC431 - Fluorochemical surfactant supplied by 3M. |
| Butvar B72 | - Polyvinylbutyral, supplied by Monsanto. |
| Paraloid B72 | - Acrylic resin supplied by Rohm & Haas. |

## Example 1

### Synthesis of Leuco Dye I

### 5-Ethylphenazinium ethosulphate

Phenazine (50g) and Diethyl sulphate (82.5g) were stirred at 145°C for 30 minutes. The mixture was left to cool and 500ml of acetonitrile were added. The resulting solution was decanted into 2 litres of ether with stirring. The product was collected by filtration washed with ether and dried. The solid which did not dissolve in acetonitile was taken up in acetonitrile and poured into ether to give additional amount of product. Yield 62.26 (67%).

5-ethyl 3-phenazinone

Ethyl phenazinium ethosulphate (62g) was dissolved in 2.5l of water and 125g of potassium ferricyanide was added. 327ml of 10% aqueous sodium hydroxide was added slowly; the solution turned dark red. After standing overnight 300ml of 40% aqueous sodium hydroxide was added. The resulting precipitate was collected by filtration washed with water and dried. Yield 36.42g (88%).

3-chloro 5-ethyl phenazinium iodide

5-ethyl 3-phenazinone (17g) was dissolved in phosphoryl chloride (80ml). Phosphorous pentachloride (15.6g) was added and the mixture stirred for two hours. The product (chloride salt) was collected by filtration, washed with ether and dried over KOH. This solid was dissolved in water (500ml), filtered to remove some insoluble tar, and the iodide was precipitated by the addition of potassium iodide (17.9g). The product was collected by filtration, washed with water and dried in vacuo. Yield 19.9g (71%).

3-(N-benzyl(-N-methyl(amino) 5-ethyl phenazinium iodide

The chloro phenazinium salt (15.5g) was dissolved in acetonitrile (2.5l), filtered and benzylmethyl amine (12.7g) was added. The mixture was stirred for 20 hours and the solvent removed by rotary evaporation. The resulting solid was dissolved in ethanol (600ml) and then reprecipitated by pouring into ether (3 litres). Yield 11.1g (58%).

Leuco Dye I

The above magenta dye (12g) was dissolved in dichloromethane (250ml) and stirred gently with a solution of sodium dithionite (12.2g) in water (250ml). A solution of benzoyl chloride (5g) in dichloromethane (10ml) was added slowly into the lower layer. The pH of the upper layer was maintained at 5 to 6. The organic layer was separated, washed with dilute aqueous sodium hydroxide, and brine. The solution was absorbed onto silica gel and rotary evaporated to dryness. The product was washed from the silica with ether. The ether solution was evaporated to yield 8.2g of Leuco Dye I.

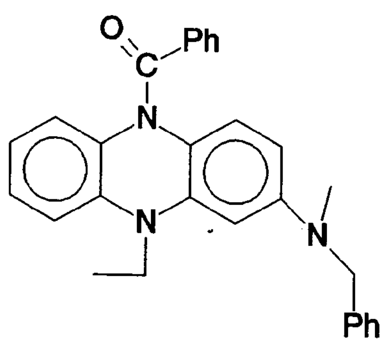

Leuco Dye I (Magenta)

(N.B."magenta" refers to the colour of the dye corresponding to Leuco Dye I. The same convention is followed in the identification of Leuco Dyes II-XI)

Example 2

Preparation of Leuco Dye II

3-morpholino-5-ethyl phenazinium iodide

5-ethylphenazinium ethyl sulphate (7.69g, 23mmol) was weighed in a 1 litre 3-necked round bottomed flask, and dissolved completely in ethanol (400ml). Air, dried by passage through concentrated sulphuric acid, was bubbled through the solution. Morpholine (2.0g, 23mmol) was then run into the stirred solution. A dye began to form immediately.

After 2 hours of air bubbling through the solution, solvent evaporation gave a tar which crystallised on standing.

The ethyl sulphate salt was dissolved in water (750ml), and the stirred solution treated with sodium iodide (250g). Stirring was continued for 0.5 hours. The precipitate was collected by suction filtration, washed with a little cold water, and then with the minimum volume of acetone, and dried in vacuo at 70°C to afford the dye (6.51g, 67%) : λmax (EtOH) 526nm (1.62 x $10^4$).

Leuco Dye II

The above dye (1.264g, 3mmol) and zinc powder (0.261g, 4mmol) were weighed into 100ml round bottomed flask, which was then sealed with a rubber septum and degassed with a stream of nitrogen. Anhydrous THF (20ml) was introduced, and the suspension was degassed for a further 0.5 hours.

Glacial acetic acid (0.223g, 4mmol) was introduced by syringe. On stirring vigorously, the colour of the reaction mixture faded to a yellow solution. After 0.5 hours, triethylamine (0.44g, 4.3mmol) was added, followed by acetyl chloride (0.77g, 10mmol).

The solution was left to stir overnight at room temperature under nitrogen.

After 19 hours, triethylamine (1.0g, 10mmol) was added by syringe. Water (10ml) was then added slowly, followed by chloroform (20ml). The mixture was exposed to air only once this point had been reached. The aqueous phase was separated, filtered under suction, and washed once with chloroform (20ml). The combined organic extracts were washed with water (3 x 25ml) until no trace of dye remained, then dried ($MgSO_4$).

Solvent evaporation gave an oil (1.117g) which crystallised on standing. Recrystallisation from EtOAc-$Et_20$ afforded pure Leuco Dye II as faintly coloured platelets (0.5g 56%) mp 161 to 162°C.

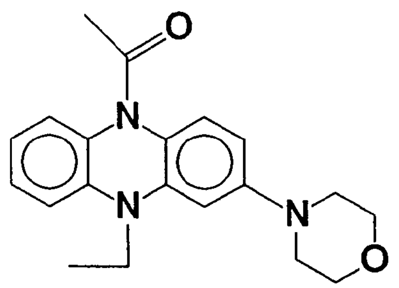

Leuco Dye II (purple)

Example 3

Synthesis of Leuco Dye III

3-dibenzylamino-5-ethyl phenazinium iodide

This compound was prepared from 3-chloro 5-ethyl phenazinium iodide of Example 1 and dibenzyl amine.

Leuco Dye III

The dye was prepared from the above dibenzyl magenta dye and benzoyl chloride in a similar manner to the preparation described for Leuco Dye I.

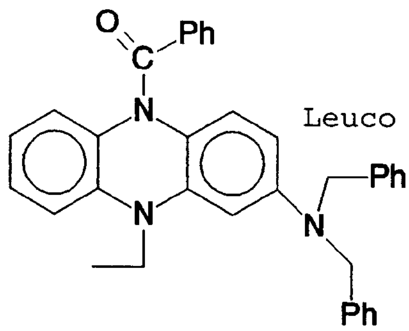

Leuco Dye III (Magenta)

Example 4

Synthesis of Leuco Dye IV

Leuco Dye III (3g) was dissolved in methanol (75ml). Palladium hydroxide (0.3g - dried in vacuo over $P_20_5$) was added and the mixture hydrogenated at atmospheric pressure for 12 hours. The solution was filtered and evaporated to give pure Leuco Dye IV (1.9g).

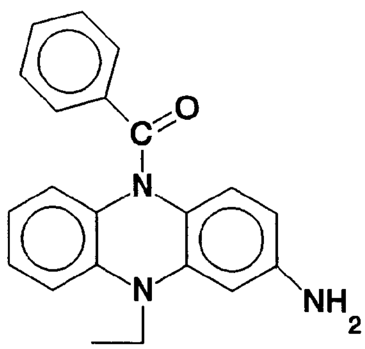

Leuco Dye IV (red)

Example 5

Synthesis of Leuco Dye V

3-(2'carboxybenzamido) 5-ethyl 10-benzoyl 5-10 dihydrophenazine

1g of Leuco Dye IV was refluxed with phthalic anhydride (0.5g) in dichloromethane for six hours. The white solid product was collected by filtration and dried. Yield 1.2g.

Leuco Dye V

The above compound was refluxed in acetic acid (10ml) containing sodium acetate (0.1g) for three hours. The solution was cooled, poured into water and the product collected by extraction into dichloromethane. The dichloromethane extract was washed with aqueous sodium bicarbonate and water, dried over $MgSO_4$ and evaporated to yield 0.81g of Leuco Dye V.

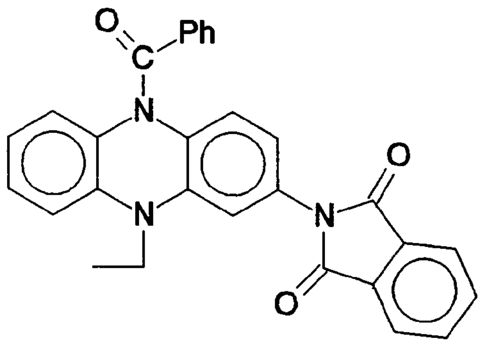

Leuco Dye V (yellow)

Leuco Dyes VI to XI were prepared by analogous methods.

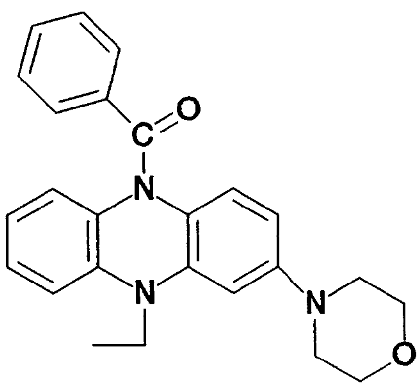

Leuco Dye VI (purple)

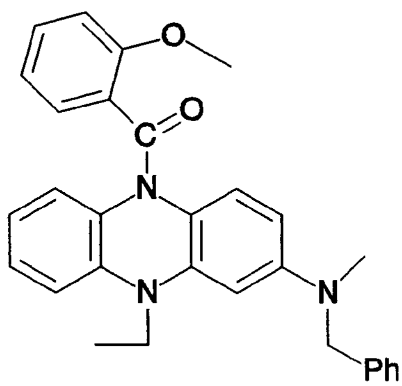

Leuco Dye VII (magenta)

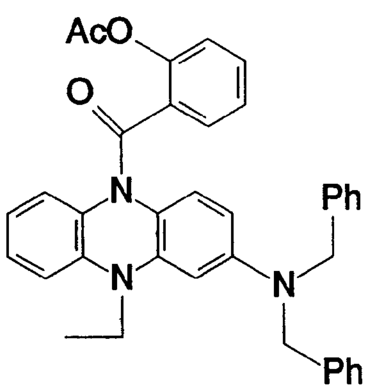

Leuco Dye VIII (magenta)

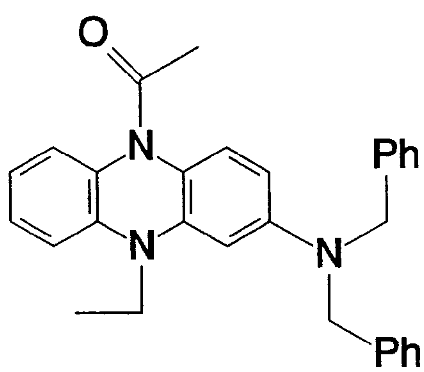

Leuco Dye IX (magenta)

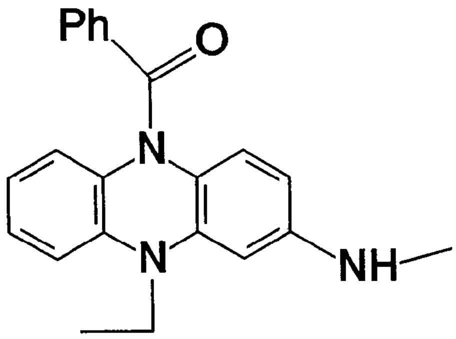

Leuco Dye X (red)

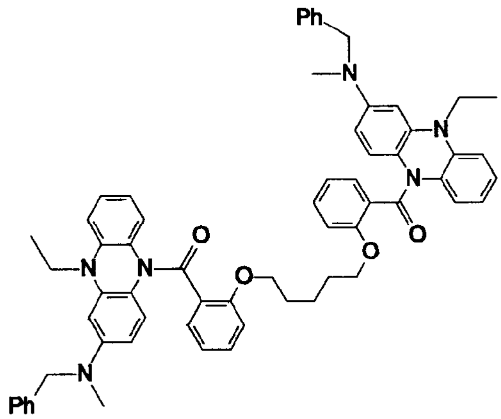

Leuco Dye XI (magenta)

In the following Examples the sensitising dyes used were:

Blue Sensitising Dye

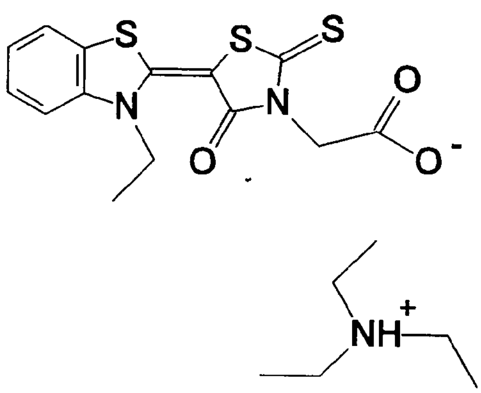

Green Sensitising Dye

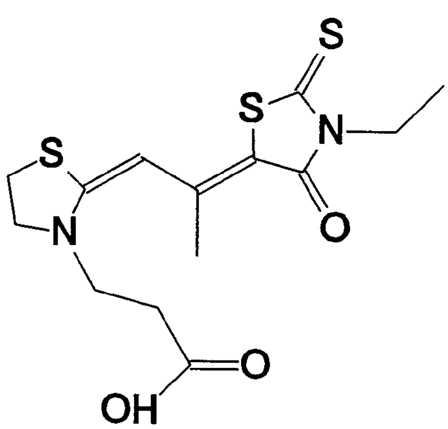

Red Sensitising Dye

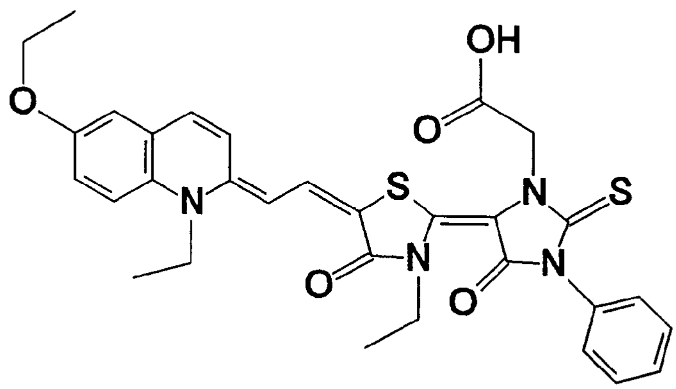

Example 6

Use of Leuco Dye in Photothermographic Systems

A dry silver formulation was prepared as follows:

| | |
|---|---|
| Halidised Behenate Soap | 10 g |
| Blue sensitiser (0.02%) | 0.5ml |
| Magenta Leuco Dye II | 0.12g |
| Methanol | 3ml |
| Toluene | 2ml |
| 4-Methylphthalic acid (4-mpA) | 0.15g |

The halidised behenate soap and the sensitiser were premixed at least 0.5 hours prior to preparation of the total formulation. An example of Soap Halidisation can be seen in Example 8.

A receptor layer of VYNS was coated from a 15% solution in MEK-toluene (1 : 1) at 75μ wet thickness on polyester base using a knife edge coater, then dried at 58°C for five minutes.

The leuco soap formulation was similarly coated onto the VYNS, then again dried at 58°C for 5 minutes.

The bilayer construction was processed by exposure to light (E,G&G sensitometer, 0 to 3 wedge, 0.001 seconds, 47B filter), cut into strips, then developed by heating at 138°C for times ranging from 1 to 18 seconds. On peeling the layers apart, a purple-magenta image was seen to have formed in the receptor layer for a development time of two seconds or more.

The corresponding general class of leuco-diaminophenazines i.e. formula I, Y = amino, cannot be formulated in this type of coating as the leuco is highly unstable to the presence of acid; it becomes necessary to coat the acid activator in a separate layer. For this reason, it is not possible to show a comparative example.

Example 7

A dry silver formulation was prepared as follows:

| | |
|---|---|
| Halidised Behenate Soap (blue sensitised as in Example 6) | 10g |
| Magenta Leuco I | 0.05g |
| Toluene | 2ml |
| Acetone | 2ml |
| 4-Methylphthalic acid | 0.05g |

A receiver layer was first coated at 100µm wet thickness and dried for 5 minutes as before.

The leuco soap formulation was coated on top of the receiving layer as before.

The material was processed as in Example 6. On peeling apart the receiving layer showed a transferred dye image.

Using VYNS as the receptor polymer gave a magenta image.

Using polyvinyl alcohol as the receptor polymer (100µm wet; 8% in $H_20$) gave a much more purple dye image.

The colour of the dye formed can therefore be varied with choice of receiving polymer.

Example 8

A model bipack construction was built up using the lamination procedure illustrated in Figure 1 of the accompanying drawings to provide the following construction:

wet thickness

| | |
|---|---|
| 100µ | Cyan soap |
| 100µ | blank |
| 100µ | Magenta soap |
| 75µ | VYNS |
| | 4 mil non-subbed opaque base |

Each layer to be laminated down was coated on clear polyester (2mil), which had been previously treated with a 1% solution of FC 431 in ethanol and dried for 1 minute at 80°C, to aid delamination.

Laminating temperatures: upper roller 260°F
lower roller 140°

Coated sheets were placed on 3M Matchprint base, with the receiving sheet i.e. opaque sheet uppermost, nearest the higher temperature roller, and passed through the lower tray of the 3M Matchprint Laminator MR-447 whilst a polyester cover sheet was fed in the upper tray.

| Soaps | | | |
|---|---|---|---|
| | Soap A | Soap B | Stir/mins. |
| 11% ½ soap homogenate in 100% EtOH | 162.5g | 212.5g | 20 |
| EtOH | 287.4g | 242.4g | |
| B72/EtOH (10% solution) | 41.8g | 41.8g | 20 |
| $HgBr_2$ (0.72g/20ml EtOH) | 4ml | 4ml | 60 |
| $ZnBr_2$ (0.45g/20ml EtOH) | 4ml | 4ml | 120 |
| Butvar B72 (solid) | 32g | 27g | 120 |
| FC 431 | 1.6g | 1.6g | 60 |
| EtOH | 10ml | 10ml | |

Formulation

| Magenta | | |
|---|---|---|
| Receptor | VYNS (15% in MEK/Toluene) | 8g |
| | 4-MPA | 0.18g |
| | FC 431 | 12 drops |

Coated at 75μm wet thickness, dry 5 minutes at 80°C.

| | | |
|---|---|---|
| Soap | Soap A | 20g stir and stand 30 |
| | Green Sensitising Dye in MeOH | 4ml to 60 mins. |
| | Magenta Leuco XI | 0.30g |
| | Paraloid B72 (16.3% in toluene) | 2g |

Add leuco/Paraloid mixture to soap, shake and coat at 100μm, dry 5 minutes at 80°C.

| Blank | |
|---|---|
| Soap A | 20 g |
| FC431 | 12 drops |

Coat at 100 μm, dry 5 minutes at 80°C.

| Cyan | |
|---|---|
| Soap B | 20g stir and stand 30 |
| Red Sensitiser (0.033% in MeOH) | 0.20 ml to 60 mins. |
| 4-MPA | 0.16g |
| EtOH | 1ml |
| cyan leuco | 0.30g |
| Toluene | 1.5ml |
| FC431 | 10 drops |

Add acid to leuco then immediately to soap, shake and coat at 100μm, dry 5 minutes at 80°C.

Expose strips to red (25) and green (58) filters on EC&G sensitometer 0 to 3 wedge for 1/1000 seconds.

Development temperature 135°C ± 2°C, for a range of times, e.g. 10, 20 .......... 60 seconds.

The processed elements exhibited:

Excellent colour separation before and after silver strips are removed.

Good magenta density in receptor (1.01 at 30 seconds); moderate cyan density in receptor (0.67 at 30 seconds).

Low Dmins: ca 0.05 to 0.09 in receptor at 30 seconds
ca 0.16 to 0.21 in full construction at 30 seconds.

The above formulations and Figures 2 and 3 of the accompanying drawings which are graphs of Reflection density versus Wavelength for Red and Green exposures show the monoaminophenazines leucos of this invention work well in multicolour systems.

Figure 2 records the results for red exposure of a cyan soap reference (curve A), the bipack with blank soap interlayer (curve B), and the bipack without an interlayer (curve C).

Figure 3 records the results for green exposure of a magenta soap reference (curve D), the bipack with blank soap interlayer (curve E), and the bipack without an interlayer (curve F).

The data shows that good colour separation is obtained, especially when there is a blank soap layer between the magenta and the cyan soaps.

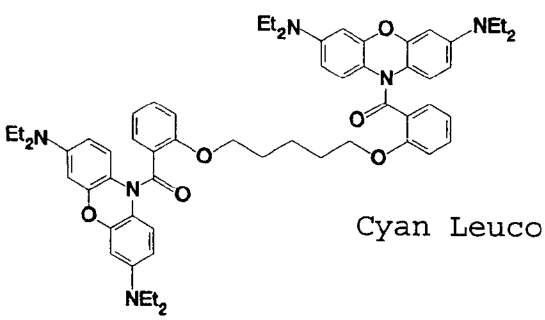

Cyan Leuco

Example 9 (Comparative)

A leuco-diaminophenazine of the structure shown below was tested in a bilayer construction similar to that of Example 6. The following formulation was coated on top of a VYNS receptor layer as described previously:-

| | |
|---|---|
| Halidised behenate soap | 10g |
| Green sensitiser (0.008% in MeOH) | 0.25mis |
| Toulene | 1.25g |
| Leuco dye | 0.1g |
| 4-MPA | 0.03g |

The bilayer construction was exposed through a green filter (No. 58) by the same method as before, and processed at 135°C for varying times. Even for very short processing times (e.g. 6 seconds), there was an unacceptable build-up of magenta dye density (fog) in the background areas. This was in spite of the fact that the concentration of acid activator 4-MPA was reduced 5-fold from the level used in Example 6, and also the fact that the leuco-diaminophenazine comprised electron-attracting trifluoromethyl substituents on the dialkylamine groups, which normally exert a stabilising effect on the leuco.

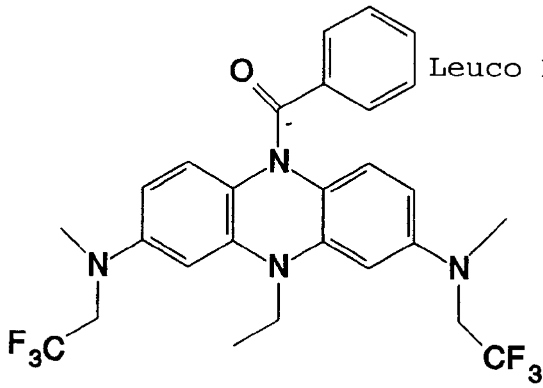

Leuco Dye

## Claims

**1.** A leuco dye having a nucleus of the general formula:

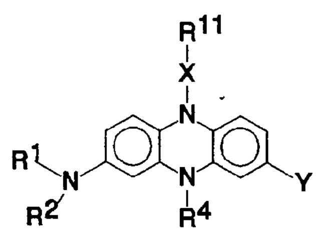

(I)

in which;

X represents

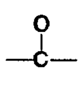 , 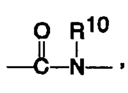 , 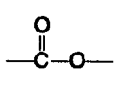 or 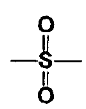

Y is H or any substituent other than amino or amido,

$R^1$, $R^2$, $R^4$ and $R^{10}$ are independently selected from H, alkyl, aryl, alkylsulfonyl, arysulfonyl, alkylcarbonyl, arylcarbonyl, or

$R^1$ and $R^2$ together represent the necessary atoms to complete a cyclic structure or one or more of $R^1$, $R^2$ and $R^4$ represent the necessary atoms to complete a cyclic structure fused to the nucleus having skeletal atoms selected from C, N, 0 and S, and

$R^{11}$ represents any group which will not prevent oxidative cleavage of the X-N bond,

with the proviso that when $R^1$ is $C_2H_5$

$R^2$ is not $C_2H_4NHSO_2CH_3$.

2. A leuco dye as claimed in Claim 1 having the general formula:

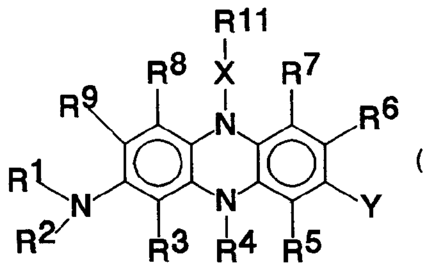

(II)

in which;

$R^1$, $R^2$, $R^4$, $R^{11}$, X and Y are as defined in Claim 1,

$R^3$ and $R^5$ to $R^9$ are independently selected from H, alkyl, aryl, heterocycic, alkoxy, aryloxy amino, cyano, hydroxy, halogen, nitro, mercapto, alkylthio, arylthio, alkylsuphonyl, arylsulphonyl, alkylcarbonyl, arylcarbonyl, acyloxy, amido, sulphonamido, and one or more of $R^2$ and R3, $R^3$ and $R^4$, $R^4$ and $R^5$, $R^5$ and Y, Y and $R^6$, $R^6$ and $R^7$, $R^8$ and $R^9$, or $R^9$ and $R^1$ may, together with the intervening skeletal atoms, complete a cyclic structure wherein the ring atoms are selected from C, N, O and S,

3. A leuco dye as claimed in Claims 1 or 2 in which $R^{11}$ is selected from alkyl, aryl and heterocyclic groups.

4. A leuco dye as claimed in any preceding Claim in which $R^{11}$ comprises one or more additional leuco dye nuclei.

5. A leuco dye as claimed in any preceding Claim in which Y is selected from the range of substituents as $R^3$ other than amino and amido.

6. A leuco dye as claimed in Claim 1 or Claim 2 having one of the following structures:

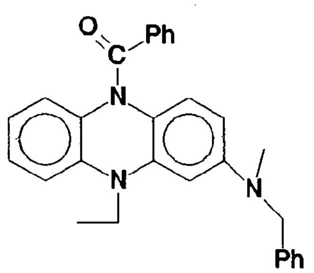

Leuco Dye I (Magenta)

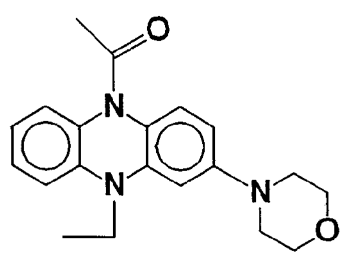

Leuco Dye II (purple)

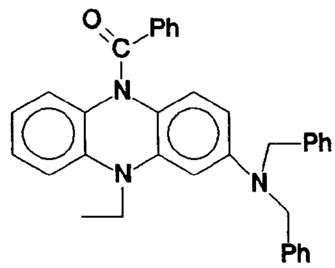

Leuco Dye III (Magenta)

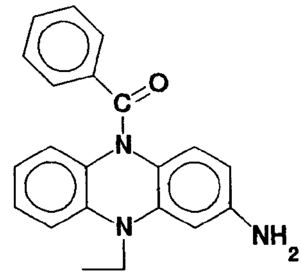

Leuco Dye IV (red)

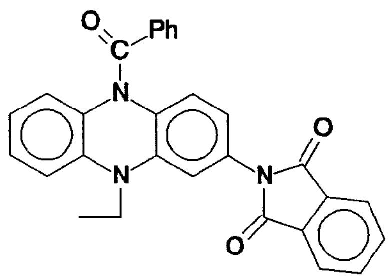

Leuco Dye V (yellow)

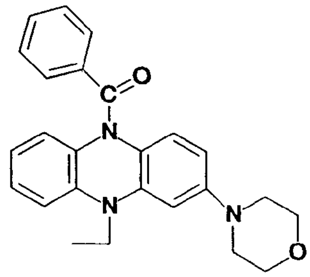

Leuco Dye VI (purple)

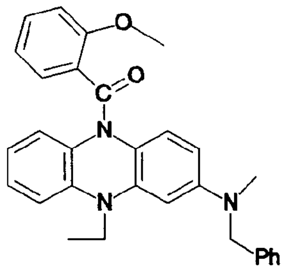

Leuco Dye VII (magenta)

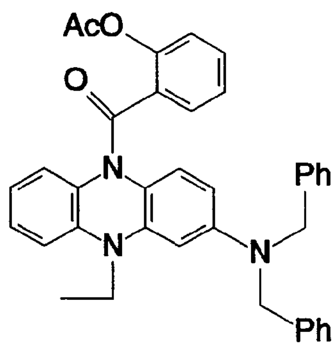

Leuco Dye VIII (magenta)

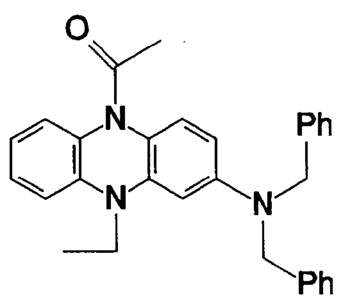

Leuco Dye IX (magenta)

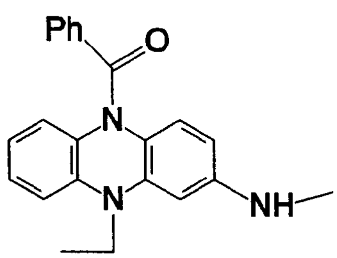

Leuco Dye X (red)

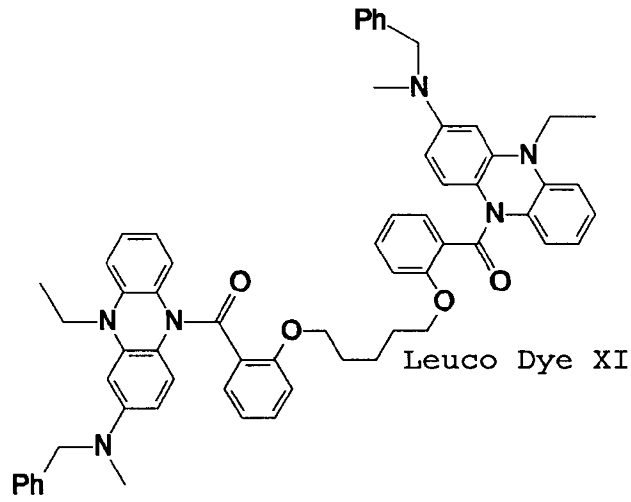

Leuco Dye XI (magenta)

7. A thermographic element comprising a silver salt oxidising agent in reactive association with a leuco dye as claimed in any one of Claims 1 to 6 or a dye of formula (I) in which $R^1$ is $C_2H_5$ and $R^2$ is $C_2H_4NHSO_2CH_3$.

8. An element as claimed in Claim 7 which is photothermographic and additionally comprises light-sensitive silver halide in catalytic association with the silver salt.

9. An element as claimed in Claim 8 comprising a plurality of photothermographic media as defined in Claim 8 in which the silver halide in the separate media is sensitised to different wavelengths and the dyes released by the leuco dyes in the separate media are of different colours.

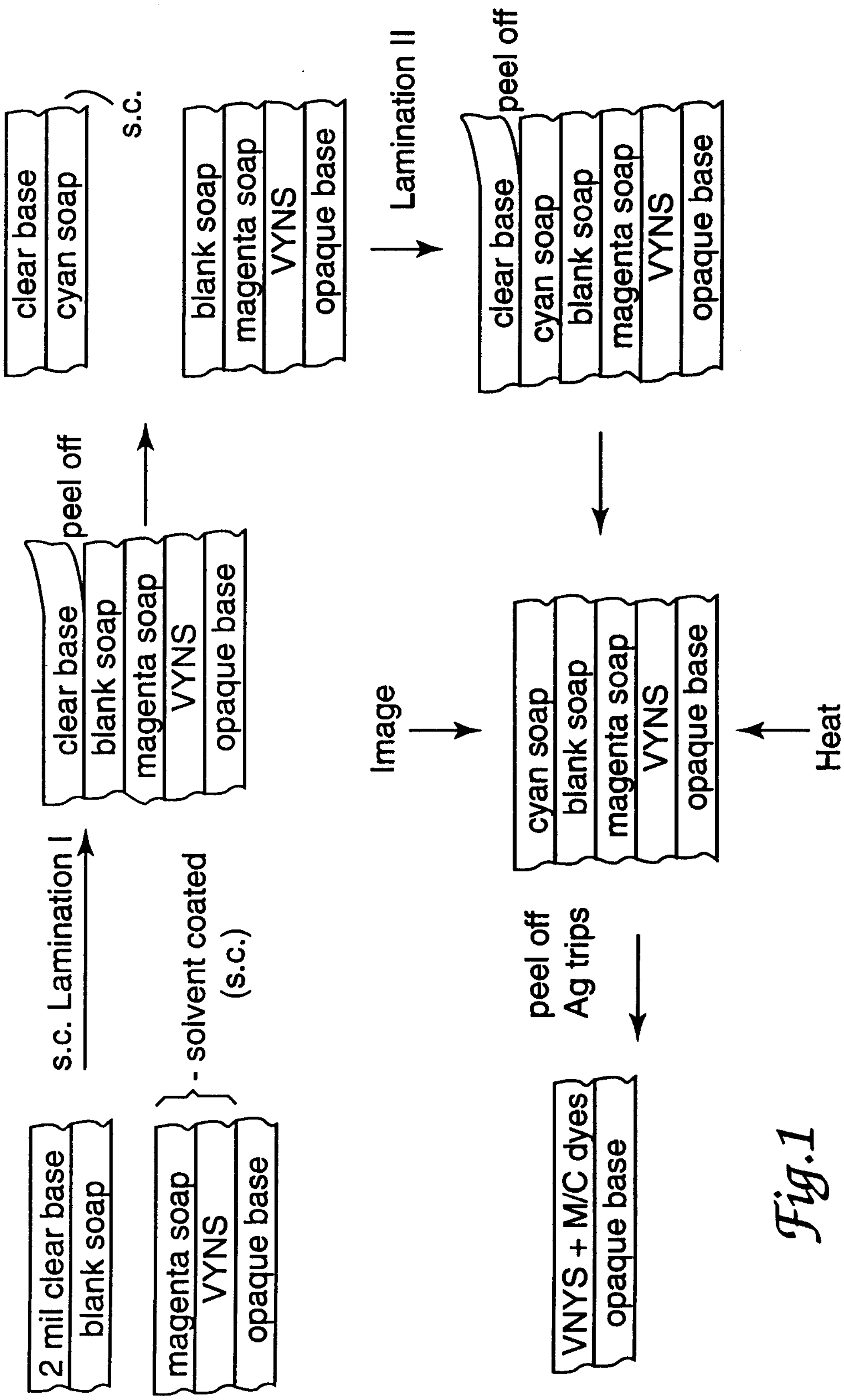
2 mil clear base
blank soap
magenta soap
VYNS
opaque base
- solvent coated (s.c.)
s.c. Lamination I
clear base
blank soap
magenta soap
VYNS
opaque base
peel off
clear base
cyan soap
s.c.
blank soap
magenta soap
VYNS
opaque base
Lamination II
clear base
cyan soap
blank soap
magenta soap
VYNS
opaque base
peel off
Image
cyan soap
blank soap
magenta soap
VYNS
opaque base
Heat
peel off
Ag trips
VNYS + M/C dyes
opaque base

Fig.1

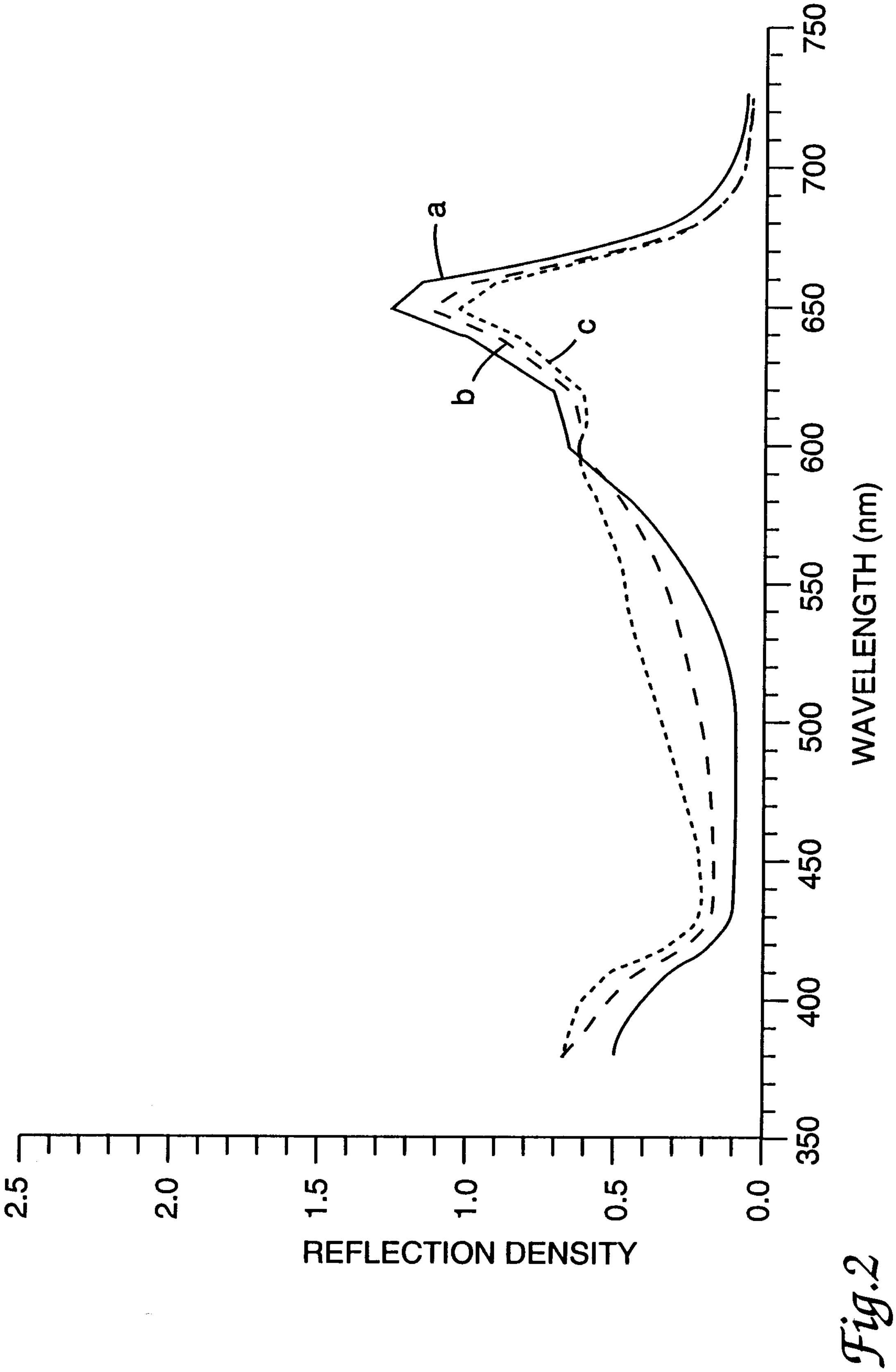
REFLECTION DENSITY
0.0
0.5
1.0
1.5
2.0
2.5
WAVELENGTH (nm)
350
400
450
500
550
600
650
700
750
a
b
c

Fig.2

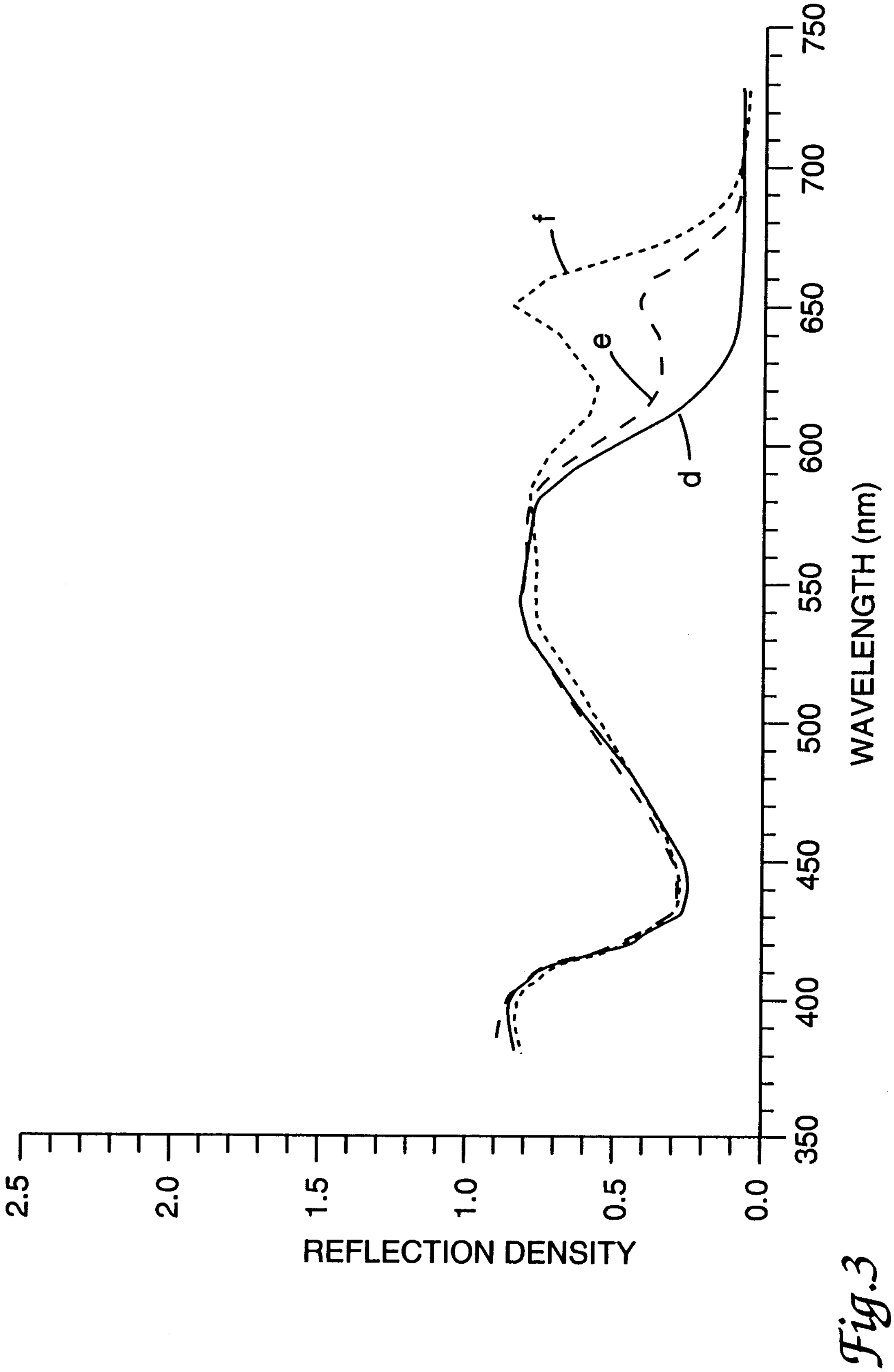
REFLECTION DENSITY
2.5
2.0
1.5
1.0
0.5
0.0
350
400
450
500
550
600
650
700
750
WAVELENGTH (nm)
d
e
f

*Fig.3*

European Patent Office

# EUROPEAN SEARCH REPORT

Application Number
EP 95 30 1483

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 75, no. 9, 30 August 1971 Columbus, Ohio, US; abstract no. 63738r, S SEREBRYANI ET AL 'Isothiocyanates of the phenazine series and N,N-9,10-diacetyldihydro-2-phenazinyl-N'-substituted -thiourea' page 451; column 1; * abstract * & UKR.KIM.ZH., vol. 37,no. 5, 1971 pages 469-472, --- | 1,2 | C07D241/46<br>C07D403/04<br>G03C1/498<br>C09B17/00<br>C09B17/02 |
| X | CHEMICAL ABSTRACTS EIGHTH COLLECTIVE INDEX , COLUMBUS, OHIO, US, page 110 * page 110 * --- | 1,2 | |
| X | CHEMICAL ABSTRACTS CHEMICAL SUBSTANCE INDEX VOL.117, COLUMBUS, OHIO, US, page 7048 * page 7048 * --- | 1,2 | TECHNICAL FIELDS SEARCHED (Int.Cl.6) |
| X | CHEMICAL ABSTRACTS CHEMICAL SUBSTANCE INDEX, VOL.117, COLUMBUS, OHIO, US, page 6485 * page 6485 * --- | 1,2 | C07D<br>G03C<br>C09B |
| X | JOURNAL OF THE CHEMICAL SOCIETY, 1958 LETCHWORTH GB, pages 4495-4498, V BARRY ET AL * the whole document * --- | 1,2 | |
| | -/-- | | |

The present search report has been drawn up for all claims

| Place of search | Date of completion of the search | Examiner |
|---|---|---|
| THE HAGUE | 22 May 1995 | Heywood, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 95 30 1483

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 53, no. 17, 10 September 1959 Columbus, Ohio, US; R ADAMS ET AL 'The preparation of aryl thioethers by reaction of aromatic halogen compounds with cuprous thiophenolate or cuprous thiobutylate' column 16145d; * abstract * & CROAT.CHEM.ACTA:, vol. 29, 1957 pages 277-285, | 1,2 | |
| D,X | EP-A-0 124 296 (MINNESOTA MINING AND MANUFACTURING) * claim 1 * & US-A-4 563 415 | 1,2 | |
| D,X | DE-A-21 54 659 (IMPERIAL CHEMICAL INDUSTRIES) * claim 1 * | 1,2 | TECHNICAL FIELDS SEARCHED (Int.Cl.6) |
| A | EP-A-0 177 328 (MINNESOTA MINING AND MANUFACTURING) * the whole document * | 1-9 | |
| A | EP-A-0 273 587 (MINNESOTA MINING AND MANUFACTURING) * the whole document * | 1-9 | |

The present search report has been drawn up for all claims

| Place of search | Date of completion of the search | Examiner |
|---|---|---|
| THE HAGUE | 22 May 1995 | Heywood, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)